# FASCICULE DE BREVET EUROPEEN

(11) **EP 1 833 307 B1**
(45) Date de publication et mention de la délivrance du brevet: **13.08.2014**
(21) Numéro de dépôt: 05826567.9
(22) Date de dépôt: 14.12.2005
(51) Int. Cl.: A23K 1/00, A23K 1/18

(54) **PROCEDE DE PREPARATION DE GRANULES DE PRINCIPE ACTIF HYDROPHILE PAR EXTRUSION**
VERFAHREN ZUR HERSTELLUNG VON PELLETS HYDROPHILER WIRKSTOFFE MITTELS EXTRUSION
METHOD FOR PREPARING PELLETS OF HYDROPHILIC ACTIVE PRINCIPLE BY EXTRUSION

(30) Priorité: 15.12.2004 FR 0413347
(43) Date de publication de la demande: 19.09.2007
(73) Titulaire: Adisseo France S.A.S., 92160 Antony (FR)
(72) Inventeur: DOLLAT, Jean-Marie, F-03100 Montluçon (FR); CHIAVAZZA, Véronique, F-69300 Caluire (FR)
(74) Mandataire: Fiorucci, Hélène
(86) Numéro de dépôt international: PCT/FR2005/003133
(87) Numéro de publication internationale: WO 2006/064127

(56) Documents cités:
- EP-A- 1 405 570
- WO-A1-03/079809
- FR-A- 2 663 818
- US-A1- 2002 068 088
- US-A1- 2004 048 814
- US-A1- 2004 173 923
- US-B1- 6 500 426

## Description

La présente invention concerne un procédé de préparation de coeurs de granulés de principe actif hydrophile. La présente invention concerne également un procédé de préparation de granulés de principe actif hydrophile destinés à l'alimentation ou au traitement des ruminants.

Certains composés, par exemple les vitamines, les sels minéraux et les aminoacides (ou acides aminés) sont essentiels dans l'alimentation des ruminants car ils sont limitants dans l'apport nutritionnel journalier. On complémente donc généralement l'alimentation des ruminants avec ces composés.

Ces substances lorsqu'elles sont administrées aux ruminants par voie orale sont détruites dans le rumen par l'action des enzymes digestives. Aussi afin d'être bénéfiques et assimilables par les animaux, ces substances sont protégées par un enrobage qui leur permet de traverser le rumen sans dommage et d'être délitées au niveau de la caillette, de façon à libérer la substance active dans l'intestin.

Il est connu de préparer des granulés adaptés à l'administration chez les ruminants et ces granulés sont généralement composés d'un coeur de substance active et d'un enrobage résistant au pH neutre du rumen et dégradable au pH plus acide de la caillette.

Une des possibilités de préparation de coeurs de principes actifs consiste à procéder à une extrusion par voie fondue. Les appareils d'extrusion sont des appareils qui utilisent à la fois la chaleur et la pression en réalisant le forçage du mélange à extruder à travers une filière. Lors de cette étape, les ingrédients actifs subissent une dégradation irréversible.

Pour résoudre ce problème, il est d'usage de chercher à modifier la composition des granulés. Par exemple, le brevet FR 2 663 818 propose l'utilisation d'un agent liant fusible. A ce titre, on a généralement recours à un corps gras, par exemple l'acide stéarique. Ces derniers présentent l'avantage de se mélanger facilement aux principes actifs hydrophobes.

Néanmoins, dès qu'il s'agit de procéder au mélange d'un principe actif hydrophile présent en une forte teneur avec l'un des agents liants fusibles connus, en se heurte à des difficultés d'homogénéisation.

La présente invention a cherché à permettre l'extrusion aisée des principes actifs hydrophiles.

Les inventeurs se sont rendus compte de manière surprenante qu'en ajoutant une étape de cobroyage des ingrédients au procédé d'extrusion, cet objectif était atteint.

Le brevet FR 2 663 818, ci-dessus discuté, ne décrit pas d'étape préalable de cobroyage des ingrédients.

La demande WO 03/079809 a trait à des pellets présentant une forte proportion de matière grasse.

La présente invention concerne donc un procédé de préparation de coeurs de granulés destinés à l'alimentation animale, lesdits coeurs comprenant :
- un principe actif hydrophile présent à une teneur active supérieure ou égale à 60% en poids,
- au moins un agent liant fusible,
- au moins un agent plastifiant,
ledit procédé comprenant (a) une première étape de mélange des ingrédients, (b) une seconde étape d'extrusion du mélange à travers une extrudeuse, notamment monovis ou bivis, munie d'une ou plusieurs filières, de manière à obtenir des joncs et (c) une troisième étape de sphéronisation des joncs, ledit procédé étant caractérisé en ce que l'on effectue un cobroyage à sec préalable des ingrédients avant l'extrusion du mélange, ledit cobroyage étant réalisé à une température au plus égale à 50°C.

Par "principe actif hydrophile", on entend toutes substances hydrophiles possédant une activité physiologique établie chez l'animal. Entrent notamment dans la catégorie de principe actif selon l'invention les compléments alimentaires. Les compléments alimentaires pour animaux sont des produits destinés à être ingérés, en complément de l'alimentation courante, afin de pallier l'insuffisance des apports journaliers en certains composés. Il est par exemple connu, de manière générale, de complémenter les rations alimentaires des animaux d'élevage avec des principes actifs, de manière à augmenter la performance zootechnique des animaux élevés. Il peut notamment s'agir de vitamines, de sels minéraux, d'acides aminés, d'oligo-éléments, d'hormones ou d'antibiotiques.

De manière avantageuse, le principe acitf actif hydrophile est un aminoacide. De manière encore plus préférée, il est choisi dans le groupe consistant en la lysine, l'arginine, la tyrosine, leurs sels et esters. A titre indicatif, le principe actif hydrophile est la L-lysine ou sa forme commerciale : le chlorhydrate de L-lysine. Il peut également s'agir du chlorhydrate de L-arginine ou du chlorhydrate de L-tyrosine.

Le principe actif hydrophile est en outre présent à une teneur active supérieure ou égale à 60% en poids du coeur de granulé. De manière avantageuse, le principe actif hydrophile est présent à une teneur active supérieure ou égale à 64% en poids du coeur de granulé.

Par "teneur active", on entend la teneur réelle en principe actif lui-même, c'est-à-dire le principe actif sous la forme de base présentant l'activité physiologique chez l'animal dont on recherche l'effet. Il peut s'agir de la forme complètement assimilée par l'organisme de l'animal. Il se peut, en effet, que les principes actifs se trouvent sous forme commerciale plus intéressante et facile à manipuler que la forme active. C'est notamment le cas quand le principe actif se trouve sous forme de sel ou analogue. Il résulte de cela que, si on décide d'utiliser une forme commerciale du principe actif qui est différente du principe actif lui-même, l'homme du métier doit calculer l'équivalence en poids, la teneur active réelle en principe actif. Cette teneur active peut, par exemple, représenter un certain pourcentage de la teneur en forme active, appartenant au mélange de composés de départ.

Le coeur de granulé comprend en outre au moins un agent liant fusible. L'agent liant fusible est sélectionné dans le groupe consistant en les cires de polyéthylène glycol, les paraffines, les huiles ou les graisses, les acides gras ayant de 10 à 32 atomes de carbone, les mono, di et triesters correspondants et les alcools correspondants. A titre indicatif, il peut notamment s'agir de l'acide stéarique. On peut également citer le Précirol^{®} et le Compritol^{®}.

Les coeurs de granulés préparés selon la présente invention comprennent également au moins un agent plastifiant. Celui-ci est préférentiellement choisi parmi la cellulose ou ses dérivés, et notamment l'éthylcellulose.

La présente invention concerne donc un procédé d'extrusion caractérisé en ce qu'il comporte une étape préalable de cobroyage à sec des ingrédients, ledit cobroyage étant réalisé à une température au plus égale à 50°C.

Par "broyage", on entend plus particulièrement l'action mécanique qui consiste à réduire les ingrédients de départ à une dimension déterminée. Le terme "cobroyage" implique le broyage de plusieurs ingrédients en même temps.

Le cobroyage est donc réalisé "à sec", c'est-à-dire que tous les ingrédients se trouvent sous forme sèche, le plus souvent sous forme de poudre. Pour réaliser le cobroyage selon la présente invention, il n'est pas nécessaire d'ajouter un ingrédient liquide au mélange, ou de mettre l'un des ingrédients, ou leur totalité, en solution.

Selon la présente invention, le cobroyage est réalisé à une température au plus égale à 50°C.

Les ingrédients constitutifs des coeurs de granulés se présentent sous forme de poudres. L'objectif de l'étape de cobroyage est de réduire les dimensions de ces ingrédients par un traitement mécanique. Le cobroyage des ingrédients avant extrusion présente en outre l'avantage d'améliorer la dispersion des constituants dans le mélange.

Le cobroyage présente donc un double intérêt : réduire la taille des ingrédients et, dans le même temps, augmenter l'homogénéité du mélange, c'est-à-dire qu'il permet également une répartition uniforme des ingrédients dans le mélange.

Selon la présente invention, les ingrédients subissent donc une étape de cobroyage avant extrusion. Le cobroyage des ingrédients nécessite l'emploi d'un broyeur, qui peut notamment être choisi parmi les broyeurs à couteaux, à rotor, à barres, à grilles, à disques ou à billes. Le choix du broyeur est principalement fonction de la granulométrie du produit broyé attendue.

Selon le présente invention, on peut également utilisé un broyeur à double enveloppe et dans lequel circule un courant d'eau, afin que le cobroyage soit réalisé à une température au plus égale à 50°C, c'est-à-dire égale à 50°C ou inférieure. Une autre possibilité consiste, par exemple, à refroidir le broyeur à l'azote liquide ou dans de la carboglace, l'objectif étant que cette étape essentielle du procédé soit réalisée à une température n'excédant pas 50°C.

Selon la présente invention, les exigences granulométriques du produit broye sont ies suivantes :

| **Taille des particules du produit broyé** | **Pourcentage dans le mélange** |
|---|---|
| Supérieur à 400 µm | 0% |
| Supérieur à 200 µm | < ou = à 15%, de préférence < ou = à 10% |
| Entre 100 et 200 µm | < ou = à 50%, de préférence < ou = à 20% |
| Inférieur à 100 µm | > ou = à 50%, de préférence > ou = à 75% |
| Inférieur à 50 µm | < ou = à 50% |

De manière avantageuse, le diamètre médian des particules du produit broyé se situe entre 50 et 100 µm. Par "diamètre médian", on entend la taille des particules du produit broyé à laquelle 50 % de l'échantillon a une taille inférieure et 50 % de l'échantillon à une taille supérieure. En l'espèce, le diamètre médian se situe entre 50 et 100 µm.

Les coeurs de granulés préparés selon la présente invention peuvent en outre comprendre un amidon. Par "amidon", on entend tout polysaccharide formé de l'association de deux polymères : l'amylose et l'amylopectine. Selon la présente invention, l'amidon peut se trouver sous forme de poudre ou sous forme d'empois. A titre indicatif, il peut s'agir d'amidon natif de blé, d'amidon natif de maïs, d'amidon natif de riz ou de fécule de pomme de terre. Il peut également s'agir des mêmes amidons traités physiquement, par exemple prégélatinisés.

On peut également ajouter aux coeurs de granulés un agent délitant qui accélère la désintégration du comprimé dans le tractus digestif. Il peut notamment s'agir de talc, de silice, de carbonate, de polyphosphate, par exemple de Na₂O, CaO, P₂O₅ ou de Al₂O₃.

Les coeurs peuvent en outre comprendre un autre principe actif.

Par "autre principe actif", on entend toutes substances possédant une activité physiologique établie chez l'animal. Entrent notamment dans la catégorie de principe actif selon l'invention les compléments alimentaires. Les compléments alimentaires pour animaux sont des produits destinés à être ingérés, en complément de l'alimentation courante, afin de pallier l'insuffisance des apports journaliers en certains composés. Il est par exemple connu, de manière générale, de complémenter les rations alimentaires des animaux d'élevage avec des principes actifs, de manière à augmenter la performance zootechnique des animaux élevés. Il peut notamment s'agir de vitamines, de sels minéraux, d'acides aminés, d'oligo-éléments, d'hormones ou d'antibiotiques.

De manière avantageuse, ledit autre principe actif est un aminoacide. A titre indicatif, on peut citer la méthionine, le tryptophane ou l'acide 2-hydroxy-4-méthylthiobutanoïque (hydroxy-analogue de méthionine) qui a l'avantage de se présenter sous forme liquide, ce qui facilite son utilisation par les sociétés productrices d'aliments. On peut également citer les sels et esters de ces composés.

Ledit autre principe actif est préférentiellement présent à une teneur active très faible, inférieure ou égale à 1 % en poids du coeur de granulé.

Selon la présente invention, le coeur de granulé comprend une teneur active en principes actifs, c'est-à-dire le principe actif hydrophile et éventuellement au moins un autre principe actif, supérieure à 64% en poids du coeur de granulé.

De façon préférentielle, l'agent liant fusible est l'acide stéarique et sa teneur dans les coeurs de granulés est au plus égale à 12% en poids de la composition des coeurs de granulés de la présente invention.

Le procédé d'extrusion selon la présente invention comprend plus particulièrement les étapes suivantes :
- mélange des ingrédients ;
- cobroyage ;
- extrusion ;
- sphéronisation ; et
- enrobage.

Les ingrédients sont d'abord mélangés, puis ils sont cobroyés.

Il est en outre possible, selon la présente invention, d'ajouter de l'eau au mélange après cobroyage et avant extrusion. A titre indicatif, on ajoute moins de 10% en poids du mélange avant extrusion d'eau. De manière préférée, on ajoute entre 3 et 5% en poids du mélange avant extrusion d'eau. Ainsi, les coeurs de granulés obtenus à l'issue du procédé sont en outre susceptibles de contenir une certaine quantité d'eau.

La granulométrie des ingrédients, suite à l'étape de cobroyage, permet l'extrusion par voie fondue des ingrédients comprenant une forte teneur en principe actif hydrophile et par conséquent un meilleur débit d'extrusion ou "extrudabilité".

La masse à extruder est ensuite forcée à travers une extrudeuse, dé préférence monovis ou bivis, munie d'une ou plusieurs filières présentant des orifices du diamètre du granulé désiré.

La qualité des extrudats est évaluée par un test de friabilité.

Après extrusion, les joncs subissent une étape de sphéronisation dont l'objet est de rendre les joncs sphériques, sans irrégularités ou aspérités de surface (le plus lisse possible).

Il est en outre important de préciser que la qualité de l'étape d'enrobage qui suit, et donc de la protection du principe actif, réside principalement dans l'étape de sphéronisation.

Dans une étape subséquente, les coeurs de granulés sphéronisés sont enrobés de manière à obtenir des granulés protégés. L'étape d'enrobage se déroule conformément à l'enseignement décrit dans les brevets EP 462 015 et EP 447 298, par une composition à base de polymère pH sensible. Cette composition présente de nombreux avantages et notamment elle n'est pas être dégradée dans le rumen mais elle est libérable dans la caillette et/ou l'intestin.

Le procédé d'enrobage comprend une première étape de polymérisation de monomères en émulsion aqueuse, une seconde étape de préparation de l'émulsion d'enrobage et une troisième étape de dépôt de ladite émulsion aqueuse sur les coeurs de principe actif.

A titre indicatif, les polymères pH sensibles, dont la préparation est réalisée par polymérisation en émulsion aqueuse, sont choisis parmi :
- les polyvinyl acétals d'esters acétylacétiques substitués par des groupes azotés dialkylés tels que le groupe diéthylamino,
- les copolymères du styrène ou de l'acétonitrile avec les isomères ou les dérivés de la vinylpyridine, et
- les sels de chitosane.

On utilise de préférence le copolymère à base de styrène et de vinyl-2-pyridine.

Le polymère est préparé par mise en contact du ou des monomères en présence d'un agent surfactant et d'un initiateur de polymérisation.

Les agents surfactants sont de préférence choisis parmi les sels alcalins d'acides gras, par exemple le sel de sodium de l'acide oléique et le sel de sodium de l'acide stéarique.

L'initiateur de polymérisation est choisi parmi les initiateurs solubles utilisés classiquement dans les procédés en émulsion, par exemple le persulfate de sodium. Le pH au cours de la polymérisation est de préférence fixé entre 10 et 14.

Une fois l'émulsion aqueuse réalisée, on prépare l'émulsion d'enrobage. On utilise alors, de préférence, comme composition d'enrobage une émulsion aqueuse contenant le polymère pH sensible obtenu à l'étape précédente, et une substance hydrophobe.

La substance hydrophobe est notamment choisie parmi les acides gras contenant 12 à 22 atomes de carbones, leurs esters et leurs sels (mono, di et triesters notamment). Il peut notamment s'agir de l'acide stéarique.

L'émulsion aqueuse peut également contenir des additifs tels que des agents antistatiques, des fongicides, des agents plastifiants, des colorants, des agents d'appétence, par exemple des additifs olfactifs, et des agents émulsifiants complémentaires.

L'émulsion est ensuite déposée sur les coeurs à enrober. Par exemple, cette émulsion est pulvérisée sur les granulés de principe actif.

Il est en outre important de préciser que, dans le cadre de la présente invention, les teneurs s'expriment en % en poids du coeur de granulé ou, selon les cas, en % en poids du granulé lui-même. De par la présence de l'enrobage, ce pourcentage diffère du pourcentage en poids du coeur de granulé et l'homme du métier doit alors calculer ce nouveau pourcentage.

Dans le cas d'un principe actif hydrophile présent à une teneur active supérieure ou égale à 60% en poids du coeur de granulé et dans l'hypothèse d'un enrobage représentant 15% en poids du granulé, le principe actif hydrophile est présent, de manière équivalente dans le granulé lui-même, à une teneur active supérieure ou égale à 51 % en poids du granulé.

Le procédé couvert par la présente invention conduit, de manière avantageuse, à des coeurs de granulés présentant une teneur active en principe actif hydrophile supérieure ou égale à 64% en poids du coeur de granulé, ou sous le même hypothèse que précédemment (c'est-à-dire un enrobage représentant 15% en poids du granulé), à une teneur active supérieure à 54,4% en poids du granulé.

Egalement, les coeurs préparés selon la présente invention peuvent comprendre un autre principe actif. Celui-ci est préférentiellement présent à une teneur active très faible, inférieure ou égale à 1 % en poids du coeur de granulé.

Dans l'hypothèse d'un enrobage représentant 15% en poids du granulé, cet autre principe actif est présent, de manière équivalente, à une teneur active inférieure ou égale à 0,85% en poids du granulé lui-même.

Les granulés de principe actif hydrophile obtenus par le procédé de la présente invention présentent de nombreux avantages. Parmi ceux-ci, et comme cela apparaît à la lecture des exemples qui suivent, figurent notamment des taux de protection et des taux de libération valorisables industriellement, et ce, alors qu'il n'est pas possible d'obtenir des granulés à forte teneur en principe actif par un procédé d'extrusion ne comprenant pas d'étape préalable de cobroyage des ingrédients.

Les conditions des tests pratiqués pour déterminer les taux de protection et de libération des principes actifs dans les granulés de la présente invention sont les suivantes :
- Taux de libération (test *in vitro)*

Il est représenté par la fraction centésimale du principe actif dissous à partir de la forme protégée, après un séjour de 2 heures sous agitation dans un milieu aqueux maintenu à pH 2 (sulfate de potassium et acide sulfurique 2N pour ajuster en permanence le pH à 2) et 40°C, dans des conditions standardisées.
A titre indicatif, le taux de libération de granulés contenant de la méthionine est mesuré dans ces conditions par iodométrie, tandis que celui de la lysine est mesuré par argentimétrie. De manière générale, on utilise l'HPLC ou toutes autres méthodes chromatographiques (échangeur d'ions notamment).
- Taux de protection (test *in vitro*)

Il représente la fraction centésimale de l'acide aminé non libéré à partir de la forme protégée, après un séjour de 24 heures sous agitation dans une solution tampon à pH 6,0 (acide phosphorique/phosphate dipotassique) et 40°C, dans des conditions standardisées.
A titre indicatif, le taux de protection de granulés contenant de la méthionine est mesuré dans ces conditions par iodométrie, tandis que celui la lysine est mesuré par argentimétrie. De manière générale, on utilise l'HPLC ou toutes autres méthodes chromatographiques (échangeur d'ions notamment). On en déduit le taux de protection par différence (différence entre quantité de principe actif introduit et quantité de principe actif libéré).

La qualité des extrudats obtenus est également évaluée. On réalise un test de friabilité, effectué sur un appareil Sotax Friabilitor USP F1 avec 10 g d'extrudats pendant 5 min à 50 rpm. La friabilité est donnée par la formule suivante : (poids initial - poids de joncs récupérés à l'issue du test) / poids initial.

L'apparence des granulés obtenus par le procédé de la présente invention, plus particulièrement leur taille et leur forme, est très importante.

Le choix de la taille des granulés dépend directement de l'application zootechnique. Elle est généralement imposée par des raisons physiologiques, par exemple pour éviter le processus de remastication chez les ruminants.

Les coeurs de granulés sphéronisés doivent être ronds, sphériques et sans aspérités (le plus lisse possible), de manière à ce que l'étape d'enrobage se déroule dans les meilleurs conditions, et à ce que le principe actif soit correctement et uniformément protégé.

Les exemples ci-dessous et tableaux permettront de mettre en évidence certains avantages et caractéristiques de la présente invention. Les inventeurs se sont rendus compte que l'extrusion d'un mélange présentant une teneur active en principe actif hydrophile, et notamment la lysine, supérieure ou égale à 60% en poids, sans avoir recours à une étape préalable de cobroyage des ingrédients avant extrusion du mélange, était rendue très difficile et risquait d'endommager le matériel.

Cette étape est donc essentielle à l'extrudabilité d'un mélange comportant une forte teneur en principe actif hydrophile.

Les exemples suivants comportent donc tous une étape de cobroyage des ingrédients avant extrusion.

### Exemple comparatif : Granulés de méthionine

895 g de méthionine, 100 g d'acide stéarique et 5 g d'éthylcellulose sont introduits dans la cuve cylindro-conique d'un mélangeur Böhle (modèle LM 40) et mélangés.

Après mélange des 3 constituants, la granulométrie du mélange est la suivante :

| **Taille des particules du produit broyé** | **Pourcentage dans le mélange** |
|---|---|
| Supérieur à 400 *µ*m | 18,1% |
| Supérieur à 200 *µ*m | 23,7 % |
| Entre 100 et 200 *µ*m | 20,7% |
| Entre 50 et 100 *µ*m | 15,8% |
| Inférieur à 50 *µ*m | 21,7% |

Le mélange est ensuite extrudé sur Bivis Haake Rheomex TW 100 configurée avec 2 vis contrarotatives (diamètre 19,7 mm et longueur 331 mm) et une filière à 9 trous (2 mm de diamètre).

L'extrudabilité est effective sur ce mélange de granulométrie relativement grossière. L'extrusion d'un mélange présentant une teneur en méthionine supérieure à 60% en poids, sans avoir recours à une étape préalable de cobroyage des ingrédients avant extrusion du mélange, est possible, a contrario des mélanges de la présente invention, c'est-à-dire de mélanges comportant une teneur active importante en principe actif hydrophile.

Le débit d'extrusion est d'environ 2 kg/h.

La friabilité des extrudats est mesurée : elle est d'environ 1,1%.

Leur densité apparente est comprise entre 0,647 et 0,648 g/cm³.

Les extrudats ainsi obtenus sont donc de qualité correcte.

En outre, un cobroyage des 3 constituants de cette formule (méthionine, acide stéarique et éthylcellulose) pour s'ajuster à la distribution granulométrique des mélanges à base de lysine couverte par la présente invention n'apporterait pas d'avantage supplémentaire. Au contraire, les inventeurs se sont rendus compte qu'en l'espèce de telles distributions granulométriques augmentent la friabilité des extrudats.

### Exemple 1 : Granulés de lysine

Le matériel utilisé est le suivant :
- un mélangeur Böhle à cuve cylindro-conique tournante modèle LM 40 ;
- un broyeur de laboratoire Retsch à grille ;
- une extrudeuse Bivis Haake Rheomex TW 100 configurée avec 2 vis contrarotatives (diamètre 19,7 mm et longueur 331 mm) et une filière à 9 trous (2 mm de diamètre) ;
- un sphéroniseur "Wyss-Probst engineering" (cuve de 300 × 100 mm) avec circulation d'huile thermostatée dans la double enveloppe ; et
- un mini lit fluide UniGlatt (cuve de 2 L équipée d'une buse "Würster").

Le test de friabilité est effectué sur un Sotax friabilator USP F1 avec 10 g d'extrudats pendant 5 min à 50 rpm.

Les conditions opératoires sont les suivantes :
800 g de chlorhydrate de lysine, 180 g d'acide stéarique et 20 g d'éthylcellulose sont introduits dans la cuve du mélangeur Böhle, et mélangés pendant 15 min à 50 rpm.

Ce mélange est ensuite cobroyé sur grille 1 mm à vitesse 1. Le cobroyage est réalisé à une température qui ne dépasse pas 50°C.

La granulométrie du mélange sortie broyeur laboratoire est telle que l'on a une poudre avec 50% de particules ayant une taille < 50 µm et moins de 10% de particules présentant une taille > 200 µm.

En sortie de broyeur, le mélange obtenu est réhomogénéisé au mélangeur Böhle pendant 15 min toujours à 50 rpm.

Ce mélange est introduit via une trémie dans l'extrudeuse Bivis dont les températures des 3 tronçons ont été préalablement réglées à :
- 72°C dans le compartiment alimentation ;
- 78°C dans le compartiment intermédiaire ; et
- 80°C dans le compartiment avant filière.

Le débit d'extrusion est d'environ 1 kg/h.

Les extrudats obtenus sont coupés à une longueur de 2 mm. Ils sont ensuite caractérisés en terme de friabilité.

La friabilité des extrudats est mesurée : elle est comprise entre 1,5 et 2%.

Les extrudats sont ensuite sphéronisés à 500 rpm, pendant 8 min et à 90°C puis tamisés entre 1,4 et 2,5 mm. Le rendement de cette opération est de 87%.

Les extrudats tamisés sont ensuite enrobés au moyen de l'émulsion préparée par agitation au polytron à une température comprise entre 75 et 90°C.

L'émulsion d'extrait sec 25% a la composition suivante :
- Acide stéarique : 20%
- Copolymère de vinyl-2-pyridine et de styrène à 20% d'ES: 24,96%
- Eau : 55%
- Soude solide : 0,04%

Le débit de pulvérisation est de 10 g/min et le rendement de 98%.

Après dépôt de l'enrobant, les extrudats sont caractérisés en terme de taux de protection et de taux de libération de la lysine.

Pour un taux d'enrobage de 14,6%, la teneur en lysine base est de 54,2%, le taux de protection mesuré *in vitro* varie entre 61 et 89%, et le taux de libération est de 95%.

| **Taux d'enrobage en %** | **Teneur en lysine base en %** | **Taux de protection *in vitro* en %** | **Taux de libération en %** |
|---|---|---|---|
| 14,6 | 54,2 | 61 à 89 | 95 |

### Exemple 2 : granulés de lysine

On reproduit l'exemple 1 avec le même appareillage mais en substituant une partie de l'acide stéarique par de l'amidon de maïs natif standard.

On mélange ainsi 800 g de chlorhydrate de lysine, 120 g d'acide stéarique, 60 g d'amidon de maïs standard et 20 g d'éthylcellulose.

Le mélange est cobroyé de façon identique à l'exemple 1.

La granulométrie du mélange sortie broyeur laboratoire est telle que l'on a une poudre avec 50% de particules < 50 µm et moins de 10% de particules > 200 µm.

Le dosage en acide stéarique (moyenne de 13 mesures) donne 11,80% pour 12 % théorique.

Le mélange est ensuite extrudé dans les conditions suivantes :
- 72°C dans le compartiment alimentation ;
- 75°C dans le compartiment intermédiaire ; et
- 78°C dans le compartiment avant filière.

Le débit d'extrusion est d'environ 2 kg/h.

On peut noter en outre que le débit d'extrusion est meilleur que celui de l'exemple 1, dans lequel l'amidon n'est pas utilisé. On peut donc envisager de doubler la productivité en utilisant de l'amidon. Ceci est une caractéristique avantageuse de la présente invention.

Les extrudats obtenus sont coupés à une longueur de 2 mm puis caractérisés en terme de friabilité.

La friabilité des extrudats est mesurée : elle est comprise entre 0,5 et 0,8%.

Il est intéressant de noter que la friabilité des extrudats comprenant de la lysine et de l'amidon est meilleure que celle des extrudats comprenant uniquement de la lysine (exemple 1).

Les extrudats sont ensuite sphéronisés à 500 rpm, pendant 6 min et à 90°C puis tamisés entre 1,4 et 2,5 mm. Le rendement de cette opération est de 88%.

Deux taux d'enrobage respectivement de 15 et 16% ont été effectués sur ce lot d'extrudats. Le débit de pulvérisation est de 10 g/min et les rendements de 98 et 97%.

Les résultats de qualité produit sont reportés dans le tableau ci-dessous.

| **Taux d'enrobage en %** | **Teneur en lysine base en %** | **Taux de protection *in vitro* en %** | **Taux de libération en %** |
|---|---|---|---|
| 14,6 | 56 | 96 | 100 |
| 16,4 | 55,2 | 99 | 100 |

### Exemple 3 : granulés de lysine

On reproduit l'exemple 2 avec le même appareillage mais en remplaçant l'amidon de maïs par de l'amidon de blé.

On mélange ainsi 800 g de chlorhydrate de lysine, 120 g d'acide stéarique, 60 g d'amidon de blé et 20 g d'éthylcellulose.

Le mélange est cobroyé dans les mêmes conditions que l'exemple 2 et extrudé dans les conditions suivantes :
- 72°C dans le compartiment alimentation ;
- 75°C dans le compartiment intermédiaire ; et
- 78°C dans le compartiment avant filière.

Le débit d'extrusion est d'environ 1,4 kg/h.

Le débit d'extrusion est également meilleur que celui de l'exemple 1, dans lequel l'amidon n'est pas utilisé.

Les extrudats obtenus sont coupés à une longueur de 2 mm puis caractérisés en terme de friabilité.

La friabilité des extrudats est comprise entre 0,6 et 1%.

Les extrudats sont ensuite sphéronisés à 500 rpm, pendant 7 min et à 90°C puis tamisés entre 1,4 et 2,5 mm. Le rendement de cette opération est de 85%.

Concernant l'enrobage, le débit de pulvérisation est de 10 g/min et le rendement est de 98 %.

Pour un taux d'enrobage de 14,6%, la teneur en lysine base est de 56,2%, le taux de protection mesuré *in vitro* est de 88%, et le taux de libération est de 100%.

| **Taux d'enrobage en %** | **Teneur en lysine base en %** | **Taux de protection *in vitro* en %** | **Taux de libération en %** |
|---|---|---|---|
| 14,6 | 56,2 | 88 | 100 |

### Exemple 4 : granulés de lysine

On reproduit l'exemple 2 avec le même appareillage mais remplaçant l'amidon de maïs par de la fécule de pomme de terre.

On mélange ainsi 800 g de chlorhydrate de lysine, 120 g d'acide stéarique, 60 g de fécule de pomme de terre et 20 g d'éthylcellulose.

Le mélange est cobroyé dans les mêmes conditions que l'exemple 2 et extrudé dans les conditions suivantes :
- 73°C dans le compartiment alimentation ;
- 75°C dans le compartiment intermédiaire ; et
- 79°C dans le compartiment avant filière.

Le débit d'extrusion est d'environ 1,5 kg/h.

Le débit d'extrusion est donc lui aussi supérieur à celui de l'exemple 1, dans lequel l'amidon n'est pas utilisé.

Les extrudats obtenus sont coupés à une longueur de 2 mm puis caractérisés en terme de friabilité.

La friabilité des extrudats est comprise entre 0,5 et 0,7%.

Les extrudats sont ensuite sphéronisés à 500 rpm, pendant 6 min et à 90°C puis tamisés entre 1,4 et 2,5 mm. Le rendement de cette opération est de 89%.

Concernant l'enrobage, le débit de pulvérisation est de 10 g/min et le rendement est de 99 %.

Pour un taux d'enrobage de 14,6%, la teneur en lysine base est de 56,2%, le taux de protection mesuré *in vitro* est de 96%, et le taux de libération est de 100%.

| **Taux d'enrobage en %** | **Teneur en lysine base en %** | **Taux de protection *in vitro* en %** | **Taux de libération en %** |
|---|---|---|---|
| 14,6 | 56,2 | 96 | 100 |

### Exemple 5 : granulés de lysine

On reproduit l'exemple 2 avec le même appareillage mais en remplaçant l'amidon de maïs par de la cellulose Arbocel.

On mélange ainsi 800 g de chlorhydrate de lysine, 120 g d'acide stéarique, 60g de cellulose Arbocel et 20 g d'éthylcellulose.

Le mélange est cobroyé dans les mêmes conditions que l'exemple 2 et extrudé dans les conditions suivantes :
- 73°C dans le compartiment alimentation ;
- 75°C dans le compartiment intermédiaire ; et
- 75°C dans le compartiment avant filière.

Le débit d'extrusion est d'environ 0,5 kg/h.

Les extrudats obtenus sont coupés à une longueur de 2 mm puis caractérisés en terme de friabilité.

La friabilité des extrudats est comprise entre 1,5 et 2%.

Les extrudats sont ensuite sphéronisés à 500 rpm, pendant 6 min et à 90°C puis tamisés entre 1,4 et 2,5 mm. Le rendement de cette opération est de 88%.

Concernant l'enrobage, le débit de pulvérisation est de 10 g/min et le rendement est de 98 %.

Pour un taux d'enrobage de 16,4 %, la teneur en lysine base est de 53,4%, le taux de protection mesuré *in vitro* est de 89%, et le taux de libération est de 100%.

| **Taux d'enrobage en %** | **Teneur en lysine base en %** | **Taux de protection *in vitro* en %** | **Taux de libération en %** |
|---|---|---|---|
| 16,4 | 53,4 | 89 | 100 |

### Exemple 6 : granulés de lysine et de méthionine

On reproduit l'exemple 2 avec le même appareillage mais en incorporant 0,35% de méthionine au détriment de l'acide stéarique.

On mélange ainsi 800 g de chlorhydrate de lysine, 3,5 g de méthionine, 116,5 g d'acide stéarique, 60 g d'amidon de maïs et 20 g d'éthylcellulose. Le mélange est cobroyé dans les mêmes conditions que l'exemple 2 et extrudé dans les conditions suivantes :
- 72°C dans le compartiment alimentation ;
- 75°C dans le compartiment intermédiaire ; et
- 78°C dans le compartiment avant filière.

Le débit d'extrusion est d'environ 1,6 kg/h.

Les extrudats obtenus sont coupés à une longueur de 2 mm puis caractérisés en terme de friabilité.

La friabilité des extrudats est comprise entre 0,4 et 0,6%.

Leur densité apparente est comprise entre 0,62 et 0,64 g/cm³.

La densité vraie calculée des granulés de 1,24 g/cm³.

Les extrudats sont ensuite sphéronisés à 500 rpm, pendant 8 min et à 90°C puis tamisés entre 1,4 et 2,5 mm. Le rendement de cette opération est de 92%.

Concernant l'enrobage, le débit de pulvérisation est de 11 g/min et le rendement est de 96 %.

Pour un taux d'enrobage de 15%, la teneur en lysine base est de 55%, le taux de protection mesuré *in vitro* est de 96%, et le taux de libération est de 100%.

| **Taux d'enrobage en %** | **Teneur en lysine base en %** | **Taux de protection *in vitro* en %** | **Taux de libération en %** |
|---|---|---|---|
| 15 | 55 | 96 | 100 |

### Exemple 7 : granulés de lysine et de méthionine

Le matériel utilisé est le suivant :
- un mélangeur industriel à ruban de 3000 litres ;
- un broyeur à disques industriel Contraplex ;
- une extrudeuse bivis industrielle Clextral modèle Evolum 53 avec 2 vis corotatives (L/D = 24), 6 fourreaux régulés à 60, 80, 80, 80, 70, 70°C de l'alimentation vers la filière, une filière droite deux fois 6 trous (longueur 12 mm, diamètre 2 mm, L/D = 6) et un couteau 4 lames ;
- un sphéroniseur "Wyss-Probst engineering" (cuve de 300 × 100 mm) avec circulation d'huile thermostatée dans la double enveloppe ; et
- un mini lit fluide UniGlatt (cuve de 2 L équipée d'une buse "Würster").

Le test de friabilité est effectué sur un Sotax friabilator USP F1 effectué avec 10 g d'extrudats pendant 5 min à 50 rpm.

Les conditions opératoires sont les suivantes :
1025 kg de mélange à 80% de chlorhydrate de lysine, 0,35% de méthionine, 11,65 % d'acide stéarique, 6% d'amidon de maïs et 2% d'éthylcellulose sont cobroyés.

La granulométrie du mélange sortie broyeur est telle que l'on obtient une poudre avec 60% de particules ayant un diamètre inférieur à 50 µm et 5% de particules ayant un diamètre supérieur à 200 µm.

L'acide stéarique dosé dans le mélange est de 12,07% (pour 11,65% théorique).

Ce mélange sert à alimenter une extrudeuse industrielle fonctionnant à 60 kg/heure (vitesse de vis 200 rpm) et à une vitesse de coupe de 1800 rpm.

Le rendement d'extrusion, défini comme le % d'extrudats de longueur supérieure à 1,4 mm, est de 99%.

La friabilité mesurée de ces extrudats est comprise entre 0,5 et 0,7%. Leur densité apparente est de 0,62 g/cm³. La densité vraie calculée des granulés de 1,24 g/cm³.

Les extrudats sont ensuite sphéronisés à 500 rpm, pendant 10 min et à 90°C puis tamisés entre 1,4 et 2,5 mm. Le rendement de cette opération est de 88%.

Pour des conditions d'enrobage identiques à l'exemple 1 et pour un taux d'enrobage de 15%, la teneur en lysine base est de 53,8%, le taux de protection mesuré *in vitro* est de 87% et le taux de libération est de 100%.

| **Taux d'enrobage en %** | **Teneur en lysine base en %** | **Taux de protection *in vitro* en %** | **Taux de libération en %** |
|---|---|---|---|
| 15 | 53,8 | 87 | 100 |

### Exemple 8 : granulés de lysine et de méthionine

On utilise le même matériel que celui de l'exemple 7 mais les extrudats sont sphéronisés et enrobés sur du matériel industriel au moyen de :
- un sphéroniseur Caleva 700 ; et
- un lit fluidisé cuve de 300 litres équipé au moyen de 5 buses en configuration top-spray.

Les conditions opératoires sont les suivantes :

On procède à l'identique de l'exemple 7.

A la sortie de l'extrusion, les extrudats sont sphéronisés par batch de 25 kg. L'appareil industriel de diamètre 700 mm est réglé à 350 rpm pour une température d'extrudats de 90°C. La durée de sphéronisation est de 12 min. Le rendement d'extrusion, défini comme le % de granulés compris entre 1,4 et 2,5 mm, est de 83%.

Les granulés sphéronisés sont ensuite enrobés dans les conditions suivantes :

| | |
|---|---|
| Quantité de granulés sphéronisés chargés : | 150 kg |
| Quantité d'enrobant pulvérisé : | 116,4 kg d'ES à 25% |
| Débit réel de pulvérisation : | soit 29,1 kg d'ES 45 kg/h |
| Quantité de granulés obtenus : | 178,8 kg |
| Quantité théorique attendue : | 179,1 kg |
| Taux d'enrobage réel : | 16,2% |
| Bilan matière : | soit 3,2% de copo V2P/Styrène 99,8% |

Pour un taux d'enrobage de 16 %, la teneur en lysine base est de 51,2%, le taux de protection mesuré *in vitro* est de 94%, et le taux de libération est de 98%.

| **Taux d'enrobage en %** | **Teneur en lysine base en %** | **Taux de protection *in vitro* en %** | **Taux de libération en %** |
|---|---|---|---|
| 16 | 51,2 | 94 | 98 |

**Tableau 1 - Tableau récapitulatif des compositions des cours de granulés**

| **Composant** | | **Exemple 1** | **Exemple 2** | **Exemple 3** | **Exemple 4** | **Exemple 5** | **Exemple 6** | **Exemple 7** | **Exemple 8** |
|---|---|---|---|---|---|---|---|---|---|
| **Lysine** | g | 800 | 800 | 800 | 800 | 800 | 800 | | |
| | % HCl | 80 | 80 | 80 | 80 | 80 | 80 | 80 | 80 |
| | % Base | 64 | 64 | 64 | 64 | 64 | 64 | 64 | 64 |
| **Méthionine** | g | Abs. | Abs. | Abs. | Abs. | Abs. | 3,5 | | |
| | % | | | | | | 0,35 | 0,35 | 0,35 |
| **Acide stéarique** | g | 180 | 120 | 120 | 120 | 120 | 116,5 | | |
| | % | 18 | 12 | 12 | 12 | 12 | 11,65 | 11,65 | 11,65 |
| **Amidon (ou équivalent)** | type | Abs. | maïs | blé | Fécule pomme de terre | Abs. | maïs | maïs | maïs |
| | g | | 60 | 60 | 60 | | 60 | | |
| | % | | 6 | 6 | 6 | | 6 | 6 | 6 |
| **Autre** | type | | | | | cellulose Arbocel | | | |
| | g | | | | | 60 | | | |
| | % | | | | | 6 | | | |
| **Ethylcellulose** | g | 20 | 20 | 20 | 20 | 20 | 20 | | |
| | % | 2 | 2 | 2 | 2 | 2 | 2 | 2 | 2 |
| **Poids total** | g | 1000 | 1000 | 1000 | 1000 | 1000 | 1000 | | |
| | ou kg | | | | | | | 1025 | 1025 |

| | | | | | | | | | |
|---|---|---|---|---|---|---|---|---|---|
| Abs. = Absent | | | | | | | | | |

**Tableau 2 - Tableau récapitulatif des caractéristiques des coeurs de granulés et granulés**

| | | **Exemple 1** | **Exemple 2** | | **Exemple 3** | **exemple 4** | **Exemple 5** | **Exemple 6** | **Exemple 7** | **Exemple 8** |
|---|---|---|---|---|---|---|---|---|---|---|
| **Amidon** | Type | Abs. | Maïs | | Blé | Fécule pomme de terre | Abs. | Maïs | Maïs | Maïs |
| **Teneur en lysine dans les coeurs** | % HCl | 80 | 80 | | 80 | 80 | 80 | 80 | 80 | 80 |
| | % Base | 64 | 64 | | 64 | 64 | 64 | 64 | | |
| **Débit d'extrusion** | kg/h | 1 | 2 | | 1,4 | 1,5 | 0,5 | 1,6 | 60 | 60 |
| **Friabilité des extrudats** | % | 1,5 - 2 | 0,5 - 0,8 | | 0,6 - 1 | 0,5 - 0,7 | 1,5-2 | 0,4 - 0,6 | 0.5 - 0,7 | 0,5 - 0,7 |
| **Taux d'enrobage** | % en poids du granulé | 14,6 | 14,6 | 16,4 | 14,6 | 14,6 | 16,4 | 15 | 15 | 16 |
| **Teneur en lysine dans les granulés** | % Base | 54,2 | 56 | 55,2 | 56,2 | 56,2 | 53,4 | 55 | 53,8 | 51,2 |
| **Taux de protection *in vitro*** | % | 61 - 89 | 96 | 99 | 88 | 96 | 89 | 96 | 87 | 94 |
| **Taux de libération** | % | 95 | 100 | 100 | 100 | 100 | 100 | 100 | 100 | 98 |

| | | | | | | | | | | |
|---|---|---|---|---|---|---|---|---|---|---|
| Abs. = Absent | | | | | | | | | | |

## Revendications

1. Procédé de préparation de coeurs de granulés destinés à l'alimentation animale, lesdits coeurs comprenant :
- un principe actif hydrophile présent à une teneur active supérieure ou égale à 60% en poids,
- au moins un agent liant fusible,
- au moins un agent plastifiant,
ledit procédé comprenant (a) une première étape de mélange des ingrédients, (b) une seconde étape d'extrusion du mélange à travers une extrudeuse, notamment monovis ou bivis, munie d'une ou plusieurs filières, de manière à obtenir des joncs et (c) une troisième étape de sphéronisation des joncs, ledit procédé étant **caractérisé en ce que** l'on effectue un cobroyage à sec préalable des ingrédients avant l'extrusion du mélange, ledit cobroyage étant réalisé à une température au plus égale à 50°C.

2. Procédé de préparation de granulés destinés à l'alimentation animale, lesdits granulés étant constitués d'un coeur de granulé comprenant :
- un principe actif hydrophile présent à une teneur active supérieure ou égale à 60% en poids,
- au moins un agent liant fusible,
- au moins un agent plastifiant,
ledit procédé comprenant (a) une première étape de mélange des ingrédients, (b) une seconde étape d'extrusion du mélange à travers une extrudeuse, notamment monovis ou bivis, munie d'une ou plusieurs filières, de manière à obtenir des joncs, (c) une troisième étape de sphéronisation des joncs, de manière à obtenir des coeurs de granulés et (d) une quatrième étape d'enrobage desdits coeurs de granulés, ledit procédé étant **caractérisé en ce que** l'on effectue un cobroyage à sec préalable des ingrédients avant l'extrusion du mélange, ledit cobroyage étant réalisé à une température au plus égale à 50°C.

3. Procédé selon l'une quelconque des revendications précédentes, **caractérisé en ce que** l'on effectue un cobroyage préalable des ingrédients de manière à ce qu'au moins 50% des particules broyées présentent une taille inférieure à 100 µm.

4. Procédé selon l'une quelconque des revendications précédentes, **caractérisé en ce que** l'on effectue un cobroyage préalable des ingrédients de manière à ce qu'au plus 50% des particules broyées présentent une taille comprise entre 100 et 200 µm.

5. Procédé selon l'une quelconque des revendications précédentes, dans lequel lesdits coeurs comprennent un ingrédient actif hydrophile sélectionné dans le groupe consistant en la lysine, l'arginine, la tyrosine, leurs sels et esters.

6. Procédé selon l'une quelconque des revendications précédentes, dans lequel lesdits coeurs comprennent en outre un amidon.

7. Procédé selon l'une quelconque des revendications précédentes, dans lequel lesdits coeurs comprennent l'acide stéarique en tant qu'agent liant fusible.

8. Procédé selon l'une quelconque des revendications précédentes, dans lequel lesdits coeurs comprennent l'agent plastifiant sélectionné dans le groupe consistant en la cellulose et ses dérivés.

## Patentansprüche

1. Verfahren zur Zubereitung von Granulatkernen, die für Tierfutter bestimmt sind, wobei die Kerne Folgendes umfassen:
- einen hydrophilen Wirkstoff, der einen wirksamen Gehalt von mehr oder gleich 60 Gew% aufweist,
- mindestens ein schmelzbares Bindemittel,
- mindestens einen Weichmacher,
wobei das Verfahren (a) einen ersten Schritt des Mischens der Bestandteile, (b) einen zweiten Schritt des Extrudierens der Mischung mit Hilfe eines Extruders, insbesondere mit einer oder mit zwei Schrauben, ausgestattet mit einer oder mit mehreren Düsen, um Stränge zu erhalten, und (c) einen dritten Schritt des Sphäronisierens der Stränge umfasst, wobei dieses Verfahren **dadurch gekennzeichnet ist, dass** eine vorherige trockene Mit-Vermahlung der Bestandteile vor der Extrusion der Mischung durchgeführt wird, wobei die Mit-Vermahlung bei einer Temperatur von höchstens gleich 50 °C durchgeführt wird.

2. Verfahren zur Zubereitung von Granulaten, die für Tierfutter bestimmt sind, wobei die Granulate aus einem Granulatkern bestehen, umfassend:
- einen hydrophilen Wirkstoff mit einem wirksamen Gehalt von mehr oder gleich 60 Gew%,
- mindestens ein schmelzbares Bindemittel,
- mindestens einen Weichmacher,
wobei das Verfahren (a) einen ersten Schritt des Mischens der Bestandteile, (b) einen zweiten Schritt des Extrudierens der Mischung mit Hilfe eines Extruders, insbesondere mit einer oder mit zwei Schrauben, ausgestattet mit einer oder mit mehreren Düsen, um Stränge zu erhalten, (c) einen dritten Schritt des Sphäronisierens der Stränge, um Granulatkerne zu erhalten, und (d) einen vierten Schritt des Umhüllens der Granulatkerne umfasst, wobei das Verfahren **dadurch gekennzeichnet ist, dass** eine vorherige trockene Mit-Vermahlung der Bestandteile vor der Extrusion der Mischung durchgeführt wird, wobei die Mit-Vermahlung bei einer Temperatur von höchstens gleich 50 °C durchgeführt wird.

3. Verfahren nach einem der vorhergehenden Ansprüche, **dadurch gekennzeichnet, dass** eine vorherige trockene Mit-Vermahlung der Bestandteile durchgeführt wird, so dass mindestens 50 % der gemahlenen Partikel eine Größe von weniger als 100 µm aufweisen.

4. Verfahren nach einem der vorhergehenden Ansprüche, **dadurch gekennzeichnet, dass** eine vorherige trockene Mit-Vermahlung der Bestandteile durchgeführt wird, so dass höchstens 50 % der gemahlenen Partikel eine Größe zwischen 100 et 200 µm aufweisen.

5. Verfahren nach einem der vorhergehenden Ansprüche, wobei die Kerne einen hydrophilen Wirkstoff umfassen, ausgewählt aus der Gruppe, bestehend aus Lysin, Arginin, Tyrosin, ihren Salzen und Estern.

6. Verfahren nach einem der vorhergehenden Ansprüche, wobei die Kerne außerdem eine Stärke umfassen.

7. Verfahren nach einem der vorhergehenden Ansprüche, wobei die Kerne Stearinsäure als schmelzbares Bindemittel umfassen.

8. Verfahren nach einem der vorhergehenden Ansprüche, wobei die Kerne, den Weichmacher umfassen, ausgewählt aus der Gruppe bestehend aus Zellulose und ihren Derivaten.

## Claims

1. A process for preparing cores of granules intended to animal feed, said cores comprising :
- a hydrophilic active principle present in an active content greater than or equal to 60 % by weight,
- at least one fusible binder,
- at least one plasticizer,
said process comprising (a) a first step of mixing the ingredients, (b) a second step of extruding the mixture through an extruder, in particular a single-screw and a twin-screw extruders, provided with one or more dies, so as to obtain rods and (c) a third step of spheronizing the rods, said process being **characterized in that** a prior dry co-grinding of the ingredients is carried out before extruding the mixture, said co-grinding being performed at a temperature at most equal to 50°C.

2. A process for preparing granules intended to animal feed, said granules being formed by a core of granule comprising:
- a hydrophilic active principle present in an active content greater than or equal to 60 % by weight,
- at least one fusible binder,
- at least one plasticizer,
said process comprising (a) a first step of mixing the ingredients, (b) a second step of extruding the mixture through an extruder, in particular a single-screw and a twin-screw extruders, provided with one or more dies, so as to obtain rods, (c) a third step of spheronizing the rods, so as to obtain cores of granules and (d) a fourth step of coating said cores of granules, said process being **characterized in that** a prior dry co-grinding of the ingredients is carried out before extruding the mixture, said co-grinding being performed at a temperature at most equal to 50°C.

3. The process according to any one of the preceding claims, **characterized in that** the prior co-grinding of the ingredients is carried out so that at least 50 % of the ground particles have a size less than 100 µm.

4. The process according to any one of the preceding claims, **characterized in that** the prior co-grinding of the ingredients is carried out so that no more than 50 % of the ground particles have a size ranging between 100 and 200 µm.

5. The process according to any one of the preceding claims, wherein said cores comprise a hydrophilic active ingredient selected from the group consisting of lysine, arginine, tyrosine, salts and esters thereof.

6. The process according to any one of the preceding claims, wherein said cores further comprise a starch.

7. The process according to any one of the preceding claims, wherein said cores comprise stearic acid as a fusible binder.

8. The process according to any one of the preceding claims, wherein said cores comprise the plasticizer selected from the group consisting of cellulose and derivatives thereof.
